(19) Europäisches Patentamt
European Patent Office
Office européen des brevets

(11) Publication number: 0 372 862 A2

# (12) EUROPEAN PATENT APPLICATION

(21) Application number: 89312578.1

(22) Date of filing: 01.12.89

(51) Int. Cl.⁵: C07K 15/00, G01N 33/68, //C12P21/00

(30) Priority: 02.12.88 US 279176

(43) Date of publication of application: 13.06.90 Bulletin 90/24

(84) Designated Contracting States: AT BE CH DE ES FR GB IT LI NL

(71) Applicant: CHILDREN'S NATIONAL MEDICAL CENTER, INC.
111 Michigan Avenue NW
Washington DC 20010(US)

(72) Inventor: Soldin, Steven J.
6335 31st Street NW
Washington DC 20007(US)

(74) Representative: Sheard, Andrew Gregory et al
Kilburn & Strode 30, John Street
London WC1N 2DD(GB)

## (54) Cyclosporine binding protein and its use in an assay for biologically-active cyclosporine.

(57) A method of quantitatively estimating biologically-active cyclosporines, analogues, derivatives and metabolites by a competitive protein binding assay uses water-soluble binding proteins isolated from the cycloplasm of human and animal cells. Tracers include radioactive atoms, fluorescent molecules, enzymes, and chemiluminescent molecules. Solution and solid phase assay methods are disclosed. Also claimed are the water-soluble binding proteins.

EP 0 372 862 A2

# CYCLOSPORINE BINDING PROTEIN AND ITS USE IN AN ASSAY FOR BIOLOGICALLY-ACTIVE CYCLOSPORINE

This invention relates to the quantitation of cyclosporine and its pharmacologically-active analogues, derivatives and metabolites in fluid samples.

Cyclosporine is a generic name for a group of neutral, highly hydrophobic cyclic undecapeptides of fungal origin (T. inflatum Gams). Cyclosporine is in widespread use as an immunosuppressant for recipients of solid organ grafts, and is effective in preventing graft-vs.-host reaction after allogeneic bone marrow transplantation. See, in general, Cohen D. J., et al., Ann. Intern. Med. 101:667 (1984).

Cyclosporine is also being investigated as a possible treatment for autoimmune diseases such as Type 1 diabetes mellitus (Assan, R., et al., The Lancet, 67 (Jan 12, 1985)), psoriasis (Ellis, C.N., et al., J. Amer. Med. Assoc., 256:3110 (1986) and vernal keratoconjunctivitis (Ben Ezra, D., et al., Transplant Proc., 20:644 (1986)).

The immunosuppressive cyclosporines and their metabolites are known to interfere with T-lymphocyte helper and effector cell proliferation, and cyclosporin A, the parent cyclosporine (CsA) selectively inhibits lymphokine production by stimulated T cells. Nonetheless, the mechanism of action of cyclosporines remains unclear. See, generally, Drugge, R.J., et al., Transplant Proc., 20:301 (1988), a review.

In view of the fact that all known regulatory biochemicals interact with a macromolecular receptor, typically a protein or glycoprotein, as an initial step in their mechanism of action, such receptors for cyclosporine have been sought. Although several candidate receptors have been identified, it is not yet settled which, if any, of these putative receptors are directly related to the mechanism(s) of action of cyclosporine. Candidate receptors for CsA include: (1) cyclophilin, a 17,628 dal. protein isolated from the soluble cytoplasmic fraction of lymphocytes, hepatocytes, thyrocytes, and other cell types [Handschumacher, R. E., et al., Science, 226:544 (1984); Harding, M. N., et al., J. Biol. Chem., 261:8547 (1986); Handschumacher, R. E., et al., U.S. Patent No. 4,722,999, issued Feb.2, 1988; Harding, M. N., et al., Adv. Inflamm. Res., 12:283 (1988) Agarwal, R. P., et al., Transplantation, 42:627 (1986)]; (2) calmodulin, a 16,680 dal. intracellular protein that mediates the effects of $Ca^{2+}$-requiring enzymes [see, in general, Hess, A.D., et al., Transplant Proc., 18:219 (1986)]; and (3) prolactin receptors on the surface T and B lymphocytes [Russell, D.A., et al., Immunol., 134:3027 (1985)].

Cyclosporin A, the first cyclosporine to be isolated, has been totally synthesized; it has a molecular weight of about 1202 daltons. All of the amino acids have the "S" configuration of the neutral L-amino acids with the exception of the D-alanine in position 8, which has the "R" configuration. Amino acids 1, 3, 4, 6, 9, 10 and 11 are N-methylated. Ten of the amino acids are known aliphatic amino acids, but amino acid 1 (also called L-9 amino acid) is a β-hydroxy amino acid. See, in general, Wenger, R.: In: White DJG (ed): Proceedings of an International Conference on Cyclosporin A, Cambridge, England, Sept. 1981, Elsevier New York, 1982, pp. 19-34. Cyclosporins A through Z are now known [Kobel, C.F. et al., Eur. J. Appl. Microbiol. Technol., 14:237 (1982)], as are a wide variety of synthetic analogues and derivatives [Rosenthaler, J., et al., published PCT application WO86/02080, published April 10, 1986].

Cyclosporine undergoes extensive metabolism in the liver, primarily through demethylation, hydroxylation and intramolecular cyclization. Maurer, G., Transplant proc., 17:19 (1985). All of the metabolites characterized to date have retained the cyclic aspect of the molecule, and, as with the parent CsA, the metabolites are extensively bound to target tissues. Ryffel, B., et al., Transplant Proc., 20:575 (1988). Some 21 metabolites of CsA are presently known, with the metabolites being designated $M_1$ - $M_{21}$; $M_1$, $M_{16}$ and $M_{17}$ are the primary metabolites of CsA.

CsA and certain of its metabolites have undesirable clinical side effects in patients when present in concentration exceeding the therapeutic drug level required for inhibition of organ rejection. Renal dysfunction commonly occurs in patients immunosuppressed with cyclosporin A [Kahan, B.D., et al., Transplant Proc., 17:1 (1985)], but it remains controversial as to whether CsA metabolites also have a nephrotoxic potential (for opposing views, see Ryffel, et al., supra). Epigastric pain, transient paraesthesia, mild hypertrichosis, gum hyperplasia, and infections are other side effects occasionally encountered. Ben Ezra, D., et al., supra.

Thus, the clinical use of cyclosporine for transplantation is complicated by the narrow therapeutic "window" between the inadequate immunosuppression that occurs at low doses and the nephrotoxicity, hepatotoxicity, and sepsis that result from overadministration. Ryffel, B., et al., Arch. Toxicol., 53:107 (1983), Myers, B.D., et al., N. Eng. J. Med., 311:699 (1984). Thus, the concentration of the CsA in plasma of transplantation patients must be monitored constantly for long periods of time. To do so, assays must be available that are capable, not only of distinguishing between CsA and its analogues, derivatives and

metabolites, but also of distinguishing between those metabolites that are pharmacologically active (as immunosuppressive or toxic agents) and those that are inert.

Currently, CsA and its metabolites are being assayed by high pressure liquid chromatography (HPLC), radioimmunoassay (RIA) using $^{3}H$ or $^{125}I$ as tracers, and fluorescence polarization immunoassay (FPIA).

Solid phase extraction of CsA and metabolites from patient samples, coupled with HPLC, have been used to separate and identify CsA and all of its metabolites. Christians, U., et al., Clin. Chem., 34:34 (1988); Lensmyer, G.L., et al., Transplant Proc., 20:614 (1988); Charles, B.G., et al., Therap. Drug Monitor, 10:97 (1988). Not only are such methods time consuming for clinical laboratory personnel, expensive to perform, and generally too slow in producing data, but they are limited to separating and estimating CsA and all of its metabolites, and are not capable of distinguishing between those that are biologically active from those that are inert. HPLC appears to be the most suitable for measurements of unmetabolized CsA. Felder, R.A., et al., Clin. Chem., 32:1378 (1986).

RIA's are being used to estimate cyclosporine and its metabolites in patient samples. The various RIA methods all involve the basic step of a competitive reaction between labeled ($^{3}H$, $^{125}I$, or a fluorescent molecule) cyclosporine and the cyclosporine analyte for binding sites on an antibody specific to these compounds. As the result of the competitive reaction, antibody-bound labeled cyclosporine will be reduced in amount by the unlabeled cyclosporine analyte in the sample. See, generally, Mahoney, W.C., U.S. Patent No. 4,727,035, Feb. 23, 1988; Quesniaux, V., et al., Immunol. Lett., 9:99 (1985); Rosenthaler, J., published PCT application W086/12080, published April 10, 1986. RIA using a polyclonal antibody measures the sum of the parent compound and only those of its metabolites that possess the requisite immuno-determinates (estimated as from 7 to 32% cross reactivity, Felder, R.A., et al., supra), only some of which may be active as immunosuppressants or toxins. Furthermore, RIA values using $^{125}I$-CsA as the tracer give discrepant results compared to those assays using $^{3}H$-CsA , for reasons not yet understood. Felder, R.A., et al., supra.

RIA's for cyclosporines and metabolites using monoclonal antibodies are also known. Quesniaux [Quesniaux, V., et al., Clin. Chem., 33:32 (1987)] developed two types of monoclonal antibodies (Mab) --one specific to CsA and another, a non-specific MAb that binds both to CsA and its metabolites. A comparison of immunoassays using polyclonal antibodies and the two MAb preparations, employing both radiolabels (RIA) and fluorescent labels (FPIA), with HPLC methods, concluded that polyclonal antibodies and non-specific MAb's (whether in an RIA or FPIA), estimate both CsA and a range of its metabolites, whereas the specific MAb and HPLC measure the CsA parent compound. Gibbons, S., et al., Transplant Proc. 20:339 (1988). Others have found that the RIA for CsA is extremely non-specific, and overestimates HPLCdetermined levels of total Cs, as liver function deteriorates in liver transplantation patients. Blyden, G.T., Clin. Pharm. Therap.,37:182 (1985); Holt, D.W., et al., Clin. Chem., 34:1091 (1988).

Thus, because of the uncertain biologic significance of the differences among CsA and CsA metabolite assays and between the cross reactivity spectra of the various tests, large correlation studies are now required in order to determine which method and matrix offers the best technique for therapeutic drug monitoring so that adverse chemical events after CsA therapy may be predicted and avoided.

Thus, a great need exists for a rapid, simple and inexpensive assay for cyclosporine and its analogues, derivatives and metabolites that not only quantifies accurately and reproducibly the concentrations of these molecules in fluid samples, but also is capable of discriminating between those species that are pharmacologically active and those that are not.

The present invention relates to a method for the quantification of cyclosporine and cyclosporine-like functional activity in fluid samples and blood cells. This method comprises a competitive protein binding assay using as the binding reagent a water-soluble cytoplasmic macromolecular cyclosporine binding protein. It is thus an object of this invention to provide water-soluble macromolecular binding proteins specific for cyclosporine and pharmacologically-active metabolites.

It is a further object to provide an aqueous solution-based competitive protein binding assay for cyclosporine and pharmacologically active metabolites using the aforementioned water-soluble binding proteins.

It is another object to provide a solid state competitive protein binding assay for cyclosporine and pharmacologically active metabolites using the aforementioned soluble binding proteins.

It is yet another object of the invention to provide cyclosporines labeled with radioactive atoms, enzymes, fluorescent molecules, or chemiluminescent molecules, suitable for use in the competitive protein binding assay of the invention.

These and other features and advantages of the invention will be apparent from the following description of the preferred embodiments thereof, and from the claims.

The description refers to the accompanying drawings, in which:-

FIGURE 1 is a standard curve obtained from the competitive protein binding assay of the invention

using [$^3$H]-dihydro CsA as the tracer and an extract (420 µg protein) of human mononuclear leukocytes as the cytosolic binding protein ;

FIGURE 2 is the same as Figure 1, but using [$^{125}$I]-CsA as the tracer ;

FIGURE 3 is the same as Figure 1, but using 120 µg of cytosolic binding proteins ;

FIGURE 4 shows the calibration of a BIO-GEL™ P-100 molecular sieve column with molecular weight markers;

FIGURE 5 shows the results of the application of the competitive protein binding assay of the invention to cytosolic protein fractions eluting from a Bio-Gel P-100 column;

FIGURE 6 shows the cyclosporine binding activities of the column fractions from Figure 5; and

FIGURE 7 compares the results of analyzing human patient blood for cyclosporines by HPLC with a binding assay carried out in accordance with the invention.

There is general agreement that the term "receptor" refers to a macromolecule capable of recognizing and selectively binding with some ligand, and which, after binding the ligand, is capable of generating some chemical or physical signal that initiates the chain of events leading to the biological response. Blecher, M., et al., "Receptors and Human Disease", Williams & Wilkins, Baltimore, 1981, Chapter 1. It is thus an important aspect of this invention that the water-soluble binding proteins used in the competitive protein binding assay have the characteristics of a natural receptor, i.e., one that carries out the initial step in the pharmacological actions of the drug cyclosporine and its analogues, derivatives and metabolites, namely, specific and reversible binding of the drug to a protein macromolecule.

Water-soluble specific binding proteins suitable for use in assays caried out in accordance with the invention are prepared from extracts of mammalian cells such as human peripheral blood lymphocytes, monocytes or leukemia cells, bovine thymus gland, human or calf spleen cells, human or animal thymoma or lymphoma cell lines, human or animal liver or hepatoma cells, or from human or animal erythrocytes. Cyclosporine binding proteins are found in diverse phylogeny such as arthropods, trematodes, molluscs, fungi, porifera and yeast. Preferred are white cells, either from human sources or from established cell lines. Most preferred are normal or tumor human mononuclear leukocytes.

The cytoplasm is defined biologically as the non-particulate, non-membraneous portion of a cell. The soluble cytoplasm (i.e., cytosol) is defined operationally as that fraction of a cell extract that remains in the supernatant fluid following centrifugation at high g-forces, i.e., greater than 20,000 x g, for at least 15 minutes. Cyclosporine binding proteins suitable for practicing this invention are found in this supernatant fluid.

The following art-recognized techniques for disrupting cells and isolating a cytosolic macromolecule are suitable in practicing this invention. Generally, isolated white cells or erythrocytes are disrupted by one of the following methods: (1) freeze-thaw cycles at low temperatures in hypotonic solution, followed by gentle homogenization in a glass/glass or TEFLON™ /glass homogenization tube: (2) brief sonication at low temperature; (3) hypotonic lysis at a low temperature, followed by repetitive forcing of the lysate through a narrow orifice (e.g., a 26 guage hypodermic needle); or (4) direct homogenization in hypotonic solution. Most preferred is a method herein a pellet of white cells is frozen at a low temperature in a hypotonic salt solution, then thawed abruptly to lyse the cells, and the lysate gently homogenized in a TEFLON™/glass homogenizer.

Following disruption of the cells by one of the aforementioned methods, the thus-disrupted preparation is fractionated by art-recognized methods to isolate cytosolic proteins. In the preferred technique, the cell homogenate is made up in sucrose (0.25-0.32 M sucrose final concentration), then centrifuged at 20,000-40,000 x g for 30-90 minutes at 4°C in vacuo; the cyclosporine receptor protein is in the supernatant fluid, and can be stored frozen (e.g., at -70°C) until ready for use. In another technique, when cells are disrupted by brief sonication while in an isotonic buffer (e.g., 0.15 M KCl, 20 mM Tris.HCl buffer, pH 7.2, 5 mM 2-mercaptoethanol), the cytosol is recovered by centrifugation at 100,000 x g at 4° in vacuo; the cyclosporine binding protein is in the cytosol, and is frozen until use. In yet another useful technique, cell debris, after adjusting the mixture to isotonicity, is removed from homogenates by a preliminary centrifugation at 500-1000 x g for 10-20 minutes, and the cytosol is obtained by centrifugation of the supernatant fluid at 100,000-150,000 x g for 30-90 minutes at 4°C in vacuo; the receptor is in the supernatant fluid. It is clear that, whatever the cell fractionation method, the cyclosporine binding proteins will be located in the water-soluble portion of the disrupted cell preparation.

The aforementioned cytosol is used as such in competitive protein binding assays carried out in accordance with the invention, or the cyclosporine binding proteins of the cytosol are partially purified and concentrated prior to use in assays. Size exclusion and affinity chromatographic techniques are preferred for the purification and concentration steps. Such fractionation techniques are disclosed in Handschumacher, R.E., et al., U.S. Patent No. 4,722,999, issued Feb. 2, 1988; Koletsky, A.J., et al., J. Immunol.,

137:1054 (1986); Harding, M.W., et al., J. Biol. Chem., 261:8547 (1986); and Agarwal, R.P., et al., Transplantation, 42:627 (1986), which are incorporated by reference to the extent that they disclose soluble cell protein fractionation methods. Membrane molecular weight cut off filters may also be used to obtain rough fractionation. Preferred are molecular sieves (e.g., SEPHADEXTM G-75 or G-100, Pharmacia Fine Chemicals, Inc., Piscataway, NJ. or BIO-GELTM P-100, Bio-Rad, Richmond, CA) that are suitable for fractionating proteins in the 3,000-150,000 molecular weight range. For the purpose of a competitive protein binding assay according to this invention, a protein fraction is deemed acceptable if: (1) the protein binds labeled cyclosporine to a statistically significant extent, based upon the method of detection, i.e., radioactivity, fluorescence polarization, chemiluminescence and the like; (2) unlabeled cyclosporine and biologically active analogues, derivatives and metabolites thereof compete with labeled cyclosporine for specific binding sites on the protein; and (3) the signal-to-noise ratio, i.e., the ratio of total binding of labeled cyclosporine to nonspecific binding of this molecule (as these terms are defined in Principles of Competitive Protein Binding Assays infra) is at least 1.1, preferably at least 1.2.

## Labeled Cyclosporines Suitable in Practicing the Invention

For the competitive protein binding assays of this invention, labeled cyclosporines are required. Cyclosporines labeled with $^3H$, $^{125}I$ or fluorescein are available commercially. [$^3H$] CsA (label positions : 95% [Abu-$^3H$]-cyclosporine and 5% ([N-methyl-$^3H$]-Sar) cyclosporine is available from Sandoz Pharmaceuticals, Hanover, N.J. or Sandoz Ltd. Basel, Switzerland. [$^3H$]-Dihydro CsA is prepared by catalytic reduction of the meBut double bond in CsA by the Wilzbach procedure with $^3H_2$ (New England Nuclear Corp., Boston, MA). [$^{125}I$]-CsA is sold by Immuno Nuclear Corp., Stillwater, MN, 55082 as part of their INCSTARTM kit. For fluorescent polarization detection methods, CsA-fluorescein tracer, suitable for use with the TDX instrument, is available from Abbott Laboratories, Abbott Park, IL. CsA can also be labeled with fluorescein by reaction with fluorescein isothiocyanate according to Mahoney, W.C., et al., U.S. Patent No. 4,427,035, issued Feb. 23, 1988. $^{125}I$-labeled-histamine-CsC can be prepared according to the method of Wong [Wong, P.Y., et al., Clin. Chem., 32:492 (1986)].

CsA, CsB, CsC and CsD and CsA metabolites for use as standards are available from Sandoz Pharmaceuticals, Hanover, N.J.

Chemiluminescent labels such as water soluble 1,2-dioxetanes that are activated by cleavage by alkaline phosphatase or $\alpha$- or $\beta$-galactosidases have been described by Bronstein [Bronstein, I.Y. et al., J. Biolumin. Chemilumin. 2:186 (1988)] and Voyta [Voyta, J. et al. Clin. Chem. 34:157 (1988)], and can be purchased from Tropix, Inc., Bedford, MA 01730 (cat. no. ED-010).

## Principles of Competitive Protein Binding Assay Using A Soluble Binding Protein

The principle of a competitive protein binding assay is based upon the following set of reactions:

```
S* + SPECIFIC REACTOR  ------->  S* - R
(F)          (R)       <- - - -   (B)
              +
              S
             ↑↓
             S-R
```

wherein S is any substance to be assayed, S* is a labeled analogue of S, R is a specific reactor (i.e, a receptor protein or glycoprotein), and $\overline{S^*-R}$ and $\overline{S-R}$ are receptor-bound labeled and unlabeled S, respectively. See, generally, Blecher, M., et al., supra.

At its broadest, therefore, in a competitive protein binding assay for cyclosporine (or a biologically active derivative, metabolite or analogue thereof) in accordance with the invention, a labeled cyclosporine (or biologically active derivative, metabolite or analogue thereof) competes with analyte for binding to a water soluble protein as described above.

Reaction of tracer amounts of an added labeled ligand, S*, with its receptor forms, under steady state conditions, a complex between the two, $\overline{S^*-R}$; isolation of this complex serves both to identify and quantify

receptors. When a competing equilibrium is set up by the addition of the unlabeled analyte, S, to form $\overline{S\text{-}R}$, less label is found in the isolated $\overline{S^{*}\text{-}R}$ complex, and this decrease is proportional to the amount of the unlabeled analyte present. In actual practice, to generate a standard curve a fixed amount of receptor protein is exposed to a fixed tracer amount of S* in the presence of zero-to-supersaturating concentration of S. The latter concentration is, ideally, several orders of magnitude greater than the Ka of specific binding, and this fraction ("nonspecific binding") is assumed to be the same for all ligand concentrations, as nonspecific binding is assumed to be a linear function of ligand concentration. After a steady-state has been reached, the $\overline{S^{*}\text{-}R}$ complex is isolate and quantified. Data are corrected for nonspecific binding by subtracting from the total binding value the amount of label in the maximally competed vessel, i.e., the one with the supersaturating concentration of S.

The standard or calibration curve is prepared by plotting

$[B_{(std)} - NSB/B_{(0\ std)} - NSB]\ 100$ vs. log [S]

wherein $B_{(std)}$ is the amount of *S bound (i.e., $\overline{S^{*}\text{-}R}$) at each concentration of standard S, $B_{(0\ std)}$ is the amount of *S bound in the absence of standard S, and NSB represents nonspecific binding at each concentration of standard S.

Standard Solutions of Cyclosporines

Standard solutions of cyclosporines for use in the assay of the invention are prepared as follows. Stock solutions of cyclosporines (typically 10-20 µg/ml but other concentrations may be appropriate), are prepared in a polar solvent completely miscible with water. A ten-fold dilution of the stock solution in drug-free whole blood, plasma or serum produces a working standard. Further dilutions in drug-free whole blood, plasma or serum are made to produce a series of diluted cyclosporine standards ranging from 0 to 2000 ng/ml. The concentration of solvent remaining in the working standard solutions are not critical as long as they are without influence on the binding reactions. Standard working solutions of cyclosporine at the highest level (e.g., 2000 µg/ml) can be stored at 4° C, but should be used within 24 hours of its preparation. Alcohol is a preferred solvent and is selected from among $C_1$ to $C_8$ primary, secondary or tertiary alkanols. Acetonitrile is also suitable as a solvent for stock solutions of cyclosporines. Most preferred in 70% aqueous ethanol.

Extraction of Cyclosporines from Fluid Samples Prior to Assay

Cyclosporine and its analogues, derivatives and metabolites in patient samples, control samples, and standards made up in drug-free whole blood, serum or plasma must be placed in a form suitable for assay. In a preferred technique, aliquots of cyclosporine-containing fluid samples are extracted with about 20 volumes of methanol, and the precipitated proteins sedimented by centrifugation. It is also suitable to extract an aliquot of whole blood with a surfactant solution. For example, 5-10 volumes of 20 mm Tris buffer, pH 8.5, containing 0.03% (v/v) Tween 20 polyoxyethylene (20 sorbitan monolaurate) is a suitable extractant (Felder, R.A., supra). Cyclosporine and metabolites can also be extracted from serum-containing samples by the method of Yee [Yee, G.C. et al., Clin. Chem., 28:2269 (1982)] using a BAKER 10TM extraction system (SPE, J. T. Baker, Phillipsburg, NJ) and small cyano disposable extraction columns (3 ml. capacity, 40 nm diam.). In another technique suitable for whole blood, a sample of whole blood is extracted with two volumes of methanol and one volume of water, the precipitated proteins are removed by centrifugation, and the supernatant fluid containing the cyclosporine filtered through SEP-PAKTM $C_{18}$ sample preparation cartridges (Waters Chromatography Division, Milford, MA) according to the method of Charles [Charles, B.G. et al., Therap. Drug Monitor, 10:97 (1988)].

Assay Procedure

1. Binding Step

Aliquots of the alcoholic extracts containing cyclosporines, analogues and/or metabolites (the sizes of these aliquots are dependent upon the label, but typically range from 0 µl to about 1000 µl when using $^{3}H$-

or $^{125}$I-labeling), are added to assay tubes and the solvent removed at a slightly elevated temperature using a gentle stream of an inert gas, typically 40°C and $N_2$ gas.

Thereafter, an aliquot of an appropriate dilution of the cytosolic binding protein preparation is added to the test tubes, and vortexed gently to allow the cyclosporine preparation to reach steady-state binding to the protein. An aliquot of labeled cyclosporine is added to the test tube and mixed. The tubes are then incubated for a period suitable for reaching steady state binding, ranging from 0 hour (control) to 16 hours, typically 1.5 hours, at a slightly elevated temperature, typically, 37°C. Nonspecific binding tubes are prepared by adding cyclosporine-free extract to the volume of buffer equal to the volume of the binding protein aliquot.

2. Separation Step

When using any detection methods other than fluorescence polarization methods which can distinguish protein-bound fluorescein-labeled cyclosporine from unbound fluorescein-labeled cyclosporine when both are together in solution (see infra), it is necessary to separate the protein-bound labeled cyclosporine from free labeled cyclosporine.

Among the separation methods useable for this purpose are:

Method A: The contents of the binding reaction mixture are diluted with ice-cold buffer, preferably about pH 7.4, the contents filtered through a glass fiber filter such as WHATMAN™ GF/B filters (Whatman Paper, Maidstone, England), then washed with ice-cold buffers; the membrane retains the protein-bound labeled cyclosporine.

Method B: This method is the same as Method A, except that filtration is carried out using a microporous filter, e.g., a nitrocellulose 0.22 µm filter (Millipore Corp., Bedford, MA) prewashed with a solution of carrier bovine serum albumin or γ-globulin to block nonspecific binding sites; protein-bound CsA is retained by the membrane.

Method C: Following dilution of the binding reaction mixture with ice-cold buffer, a suspension of charcoal particles coated with a carrier protein (albumin or γ-globulin) is added to the tube, the mixture vortexed, then centrifuged in the cold to sediment the charcoal particles. The supernatant fluid oontains the protein-bound labeled cyclosporine.

Method D: Following dilution of the binding reaction mixture with ice-cold buffer, a suspension of polyethylene glycol particles (M.W. 15,000-20,000), e.g., 1.0 ml of a 30 mg/ml suspension, plus a solution of a carrier protein, preferably about 1.0 mg of serum albumin or γ -globulin, are added, and the resulting suspension is mixed. The sediment is collected by centrifugation and the supernatant fluid discarded. The pellet contains the protein-bound labeled cyclosporine.

Method E: Following dilution of the binding mixture with ice-cold buffer, carrier albumin or γ-globulin is added to the tube, and trichloroacetic acid added to a final concentration of about 5-10% at ice-bath temperature to precipitate all proteins. The precipitate is centrifuged and the supernatant fluid discarded. The pellet contains the protein-bound labeled cyclosporine.

Method F: Protein-bound labeled cyclosporine is separated from the unbound species in the binding mixture using minicolumns of a molecular sieve matrix, such as LH-20 SEPHADEX™ (Pharmacia Fine Chemicals, Piscataway, NJ). Washing the column with a small volume (e.g., 0.5 ml) of phosphate-buffered saline, preferably about pH 7.4, will elute, in the void volume the protein-bound labeled cyclosporine. SEPHADEX LH-20 is a weakly hydrophobic matrix, and free labeled cyclosporine will be retarded in such a matrix.

Quantification of Receptor-Bound Radioactive Cyclosporine

When $^3$H is the tracer in Methods A and B, the filters are placed in liquid scintillation counting (LSC) vials, and an aliquot of an aqueous-organic solvent phase combining scintillation system (e.g., PCSS, Amersham, Arlington Heights, IL) is added. The vials are vortexed, and the amount of radioactivity quantified by liquid scintillation spectrometry (LSS).

When using Method C, an aliquot of the supernatant fluid is added to LSC vials, diluted with, e.g. PCSS, vortexed, then counted by LSS. When using Methods D and E, the pellet is resuspended in PCSS, or dissolved in NaOH and diluted in PCSS, added to LSC vials, then counted by LSS. When using Method F, an aliquot of the void volume is diluted in PCSS and added to LSC vials, then counted by LSS. When $^{125}$I is the tracer, the filters from Methods A and B, the supernatant fluid in Method C, or the pellets from Methods

D and E, or the void volume from Method F, are placed in a glass tube, and the radioactivity is quantified in a gamma counter.

Competitive Protein Binding Assay by Fluorescence Polarization

Displacement experiments using $^3$H-labeled CsA and fluorescein-labeled CsA have demonstrated that both molecules compete for the CsA binding sites on a cytosolic cyclosporine binding protein. This observation provides the basis for using fluorescence polarization measurements to determine the amount of cyclosporine bound to its receptor protein in the competitive protein binding assay.

The principle underlying fluorescence polarization assays is described in Robbins, et al. [Robbins, B.A., et al., J. Clin. Lab. Anal., 2:62 (1988)] and in the Abbott Laboratories TDX Instruction Manual. In brief, in this assay, a beam of plane-polarized light is used to excite the fluorophore (e.g., fluorescein), and the resulting polarized fluorescent signal is measured. The assay depends on the principle that molecules in solution randomly move and rotate at a rate that is inversely proportional to their size. Small molecules (e.g., CsA-fluorescein) rotate freely and rapidly, whereas large molecules (e.g., protein-bound CsA-fluorescein) will not rotate freely, or as freely.

In the fluorescence polarization binding protein-based assay carried out in accordance with this invention, CsA-fluorescein will not produce a polarized fluorescent signal as this molecule rotates freely, whereas the same molecule bound by the cyclosporine binding protein will give a polarized fluorescent signal as it is not free to rotate. That is, polarization increases as molecular size increases.

The assay system thus involves a standard competitive protein binding assay with incubation of an initial sample containing analyte (herein cyclosporine, its derivatives, analogues, or metabolites), fluorescent-labeled drug (e.g., CsA-fluorescein), and water-soluble cyclosporine binding protein. The intensity of the polarized fluorescent signal is inversely related to analyte concentration. Dadliker, W.B., et al., Methods Enzymol. 48:380 (1978). Therefore, if a patient sample contains a low concentration of the cyclosporine analyte, after the competitive binding reaction reaches steady state there will be a high concentration of bound tracer (e.g., CsA-fluorescein) in the reaction mixture, and polarization will be high. Conversely, if there is a high concentration of cyclosporine analyte in the patient sample, after the competitive binding reaction attains a steady-state, there will be a low concentration of bound tracer in the reaction mixture and polarization will be low. This method is most useful for measurement of small molecules, which produce the greatest change in polarized fluorescence when the labeled molecule is bound to a receptor.

For the purposes of competitive protein binding assay carried out in accordance with this invention, it is an important feature of the fluorescence polarization technique that protein-bound and unbound cyclosporine analyte can be distinguished in a single reaction mixture, i.e., without the need to separate the two components.

Abbott Laboratories has adapted the fluorescence polarization system to assays of multiple therapeutic drugs in its TDX System. The TDX system for the immunoassay of cyclosporine contains, in addition to the automated TDX instrument, a metabolite reagent pack containing, in separate vials, a buffer-surfactant solution, a solution of anti-CsA antibody containing a protein stabilizer, and CsA-fluorescein in a solution containing a surfactant and protein stabilizer.

This TDX system is adaptable for a competitive protein binding assay carried out in accordance with this invention for cyclosporine and cyclosporine-like compounds by replacing the antibody vial with one containing cytosolic water-soluble cyclosporine binding proteins of the invention, and a protein stabilizer.

Cyclosporine standards, controls, and patient samples are placed in individual cartridges of the Abbott TDX instrument. The metabolite reagent pack is placed in the instrument. Thereafter, in an automated series of steps, standards, controls and patient samples are mixed with water-soluble binding protein and CsA-fluorescein, the mixtures are incubated at the preset temperature for a selected period until binding steady state is reached. The mixtures are transferred to glass cuvettes, and the fluorescent polarization signal measured. As noted above, the intensity of this signal is inversely related to the concentration of the analytes.

The fluorescent signals from patient samples are converted to $B/B_0$ ratios and these ratios are read off of a standard curve obtained by analyzing by fluorescence polarization a series of cyclosporine standards (see Standard Solution of Cyclosporines, supra), wherein the co-ordinates for the standard curve are:

$[B_{(std)}/B_{(0\ std)}]100$ Vs. log [Cs]

and $B_{(std)}$ is the fluorescence polarization of a bound standard CsA-fluorescein comples, $B_{(0\ std)}$ is that of a control sample and [Cs] is the concentration of CsA at each point.

## Quantification of Protein-Bound Alkaline Phosphatase-Conjugated Cyclosporine by Chemiluminescence

In separation Methods A and B supra, the filters are placed on a sheet of WHATMAN blotting paper. The filter is then soaked with a solution (500-1,000 µg) of 3-(2′-spiroadamantane)-4-methoxy-4-(3″-phosphoryloxy)phenyl-1,2-dioxetane, disodium salt (AMPPD, Tropix Inc., Bedford, MA) in an alkaline buffer containing $MgCl_2$. The filters are transferred to a piece of MYLARTM polyester film, and then to a black box containing instant film, such as Type 612 POLAROIDTM film. After exposure of the film to the light for an appropriate period, the dark image is digitized using, for example, a black and white RBP Densitometer, Tobias Assoc., Inc., Ivyland, PA.

In separation Method E, the pelleted suspensions are washed with pH 7.4 buffer, and once with an alkaline buffer (pH 7-10) containing $MgCl_2$. The pellets are then reacted with AMPPD in an alkaline buffer (pH 7-10) containing $MgCl_2$ until maximum luminescence is attained, typically in 15 to 30 minutes 10 at 30°C. Thereafter, the luminescence from each tube is read in a luminometer, e.g., TURNERTM 20E Luminometer or BERTHOLD CLINILUMATTM Luminescence Analyzer.

In separation Methods C and F, the supernatant fluid or the void volume respectively, are reacted with AMPPD at an alkaline pH (pH 7-10) in the presence of $MgCl_2$. After maximum chemiluminescence has been attained, typically in 15 to 30 minutes at 30°C, the luminescence is estimated in a luminometer.

Where the cyclosporine is conjugated to an α- or β-galactosidase, 3-(2′-spiroadamantane)-4-(3″-O-galactopyranoside)phenyl-1,2-dioxetane will be the substrate.

## Presentation of Competitive Protein Binding Assay Data

Standard or calibration curves are drawn for known cyclosporine standard solutions by plotting

$[Bound_{(std)}\text{-}NSB/Bound_{(0\ std)}\text{-}NSB]100\%$ vs log [CsA]

wherein the components are as defined above.

Thereafter, radioactivity, fluorescence polarization or chemiluminescence values for controls and unknowns are converted to

$[Bound_{(unknown)}/Bound_{(0)}]100$

wherein $Bound_{(unknown)}$ is the quantitative value of protein-bound cyclosporine and $Bound_{(0)}$ is the appropriate control value, by standard calculations well known in the art. The calculated ratio is then referenced to the standard or calibration curve for the determination of the concentration of cyclosporine or compounds with cyclosporine-like biological activity in the unknown samples.

## Solid-state competitive Protein Binding Assay

A supporting matrix, e.g., the bottoms of wells of a microtitre plate or the walls of a plastic tube, is coated with a cyclosporine binding protein and nonspecific binding sites are blocked by brief exposure to drug-free serum.

An aliquot of diluted alcoholic extracts of labeled CsA is contacted with the coated surface, incubated with gentle shaking, the solid surface washed with cold PBS, and the wash fluids aspirated to waste.

Thereafter, an aliquot of a patient fluid sample extract containing cyclosporine and/or its biologically active analogues, derivatives and metabolites is contacted with a protein-coated surface and incubated with gentle shaking for a suitable period ranging from 0 hours (control) to 16 hours (analyte). The incubation fluid is aspirated to waste, and the solid surface is washed gently with cold PBS.

Protein-bound labeled CsA is extracted from the solid surface by surfactant or an alcohol, as described above for extraction of cyclosporines from fluid samples. The precipitated proteins are removed by brief centrifugation, and the amount of radioactivity in the supernatant fluid quantified as described above.

In order that those skilled in the art can more-fully understand this invention, the following examples are set forth. These examples are given solely for illustrative purposes, and should not be considered as expressing limitations unless so set forth in the appended claims.

## EXAMPLE 1

Isolation of Cytosolic Cyclosporine Binding Protein

Leukopacks (obtained from the Blood Bank, Children's Hospital National Medical Center, Washington, D.C.) with a plasma-buffy coat volume of about 100 ml were diluted 3:5 (v/v) with phosphate-buffered saline, pH 7.4, (PBS) at 37°C. Diluted cells were layered over Histopaque-1077 (Sigma Diagnostics, St. Louis, MO) 3:8 (v/v) and centrifuged at 400 x g for 30 minutes at 25°C. The upper layer of diluted plasma was aspirated to waste, and the white cell layer mononuclear leukocytes at the Hypaque-plasma interface was carefully aspirated and retained. The button of erythrocytes and granulocytes was discarded.

The white cells were frozen at -70°C and thawed to lyse the cells. Further cellular disruption was accomplished by gentle homogenization with a TEFLON™ pestle in a glass tube at 5 strokes per minute for 2 minutes. The homogenate was made to 0.32 M with sucrose, then centrifuged at 20,000 x g for 40 minutes at 0-4°C. The supernatant fluid, which contained cytosolic binding proteins for cyclosporine, was stored at -70°C until needed.

Protein concentrations of the cytosol, as determined by using bicinchoninic acid protein assay reagent (Pierce, Rockford, IL), ranged from 2.1 to 4.2 mg/ml.

EXAMPLE 2

Competitive Protein Binding Assay for Cyclosporine Using the Cytosol From Example 1

1. Standard CsA solution

A 1.6 µg/ml standard was prepared by adding 0.1 ml of 160 µg/ml CsA stock solution in 70% aq. EtOH to 9.9 ml of whole blood, plasma or serum. The 1.6 µg/ml standard solution was doubly diluted in whole blood, plasma or serum to produce standards of 25, 50, 100, 200, 400, 800 and 1600 ng/ml. As controls, drug-free whole blood, plasma or serum were subjected to the same double dilutions.

2. Extraction of standards, controls or patient samples

Aliquots (50 µl) of CsA standards in whole blood, serum or plasma, controls, and patient samples were added to 950 µl of methanol, the capped tubes were mixed by vortexing for 30 seconds, and the mixture centrifuged at 2000 x g for 5 minutes to remove precipitated proteins. Cyclosporine-free whole blood was extracted as above and used as the zero standard.

3. Assay procedures

Aliquots (50 µl to 850 µl of the ethanolic extracts of step 1 were added to glass test tubes, and the solvent removed at 40°C under a gentle stream of $N_2$.

To the dried samples was added cytosolic binding protein (from Example 1) and the tube contents mixed by vortexing. An aliquot of an appropriate dilution of tracer [$^{125}$I]-CsA or [$^{3}$H]-dihydro CsA was added to each tube, and the solutions mixed by vortexing. The binding mixtures were incubated at 37°C for 12 hours. Non-specific binding tubes (NSB) were prepared by adding CsA-free extract to a volume of buffer equal to the volume of the receptor aliquot.

After the incubation period, tubes were immersed in an ice-water bath and 3.0 ml of Tris-HCl buffer (pH 7.4, 4°C) was added as a diluent. The contents of the tubes were then filtered through WHATMAN GF/B glass filters, and the filters washed once with 6.0 ml of ice-cold buffer. The filters were then sucked dry over a vacuum manifold.

When $^{3}$H was the tracer, the filters were separately placed in LSC vials and an aliquot of PCSS added. The vials were vortexed, allowed to set for 2 hours, and the radioactivity quantified by LSS. When $^{125}$I was the tracer, the filters were separately placed in glass tubes, and the radioactivity quantified by a gamma counter. The times of counting were selected so as to accumulate statistically significant numbers of counts.

Standard curves were prepared by plotting $[B_{(std)}\text{-NSB}/B_{(0\ std)}\text{-NSB}]100\%$ against the concentrations of CsA in ng/ml.

The curve of Fig. 1 was generated by a competitive protein binding assay at a fixed concentration of cytosolic receptor protein and $[^3H]$-dihydro CsA, and doubling concentrations of CsA. The standard curve was useful between about 10 ng/ml and 400 ng/ml of the cyclosporine.

The curve of Fig. 2 was generated by a competitive protein binding assay carried out in the same manner as that from which Fig. 1 was generated, but using $^{125}I$ as the tracer and plotting bound radioactivity against concentration. The CsA standard which represented 12 ng/ml in whole blood had a bound value of 12%. The CsA standard which represented 25 ng/ml in whole blood had a bound value of 2%. The striking difference between Figs. 1 and 2 indicates a greater affinity to the receptor sites for the $^{125}I$-CsA compared to $[^3H]$-dihydro CsA, and a consequently greater sensitivity. Concentrations of cyclosporine activity in whole blood equivalent to as little as about 5 ng/ml CsA can be quantified by this method when using the $^{125}I$ tracer.

## EXAMPLE 3

Competitive Protein Binding Assay for Cyclosporine

The following assay was performed as in Example 2, but using the modifications described herein below.

The volume of CsA methanolic extract of standards controls and patient samples was 800 µl. The volume of cytosolic binding protein was 200 µl of a 1:5 (v/v) dilution in buffer (0.6 mg/ml). The radioligand was $[^3H]$-dihydro CsA (25 µl). The incubation period was 12 hours. The reaction was stopped by adding 2 ml of ice-cold buffer to each reaction tube, and tubes were maintained in an ice bath for 5 minutes. Protein-bound $[^3H]$-dihydro CsA was separated from the free ligand by the glass filter method. Filters were counted in 10 ml of PCSS scintillant, and a standard curve was prepared as previously described for $^3H$ tracers.

As shown in Figure 3, a classical sigmoid relationship was obtained when % $B/B_0$ was plotted against the log of CsA in ng/ml. This is within the range of cyclosporine concentration in patients as determined by radioimmunoassay (Mahoney, W.C., U.S. patent Serial No. 4,727,035).

## EXAMPLE 4

Fractionation of Human Mononuclear Leucocyte Cytosolic Proteins

A 1 meter x 2 cm I.D. column of Bio-Rad's BIO-GEL™ P-100 molecular weight exclusion gel (fractionation range approximately 5,000-100,000 dal.) was equilibrated with 50 mM Tris. HCl buffer, pH 7.4, with 0.4 mM $CaCl_2$, 0.5 mM EDTA and 80 mM NaCl for 24 hours. The column was standardized (Fig. 4) using Pharmacia's molecular weight standards ranging between 15,000 and 66,000 dal.

A 2 ml aliquot (2.2 mg protein) of the cytosol from Example 1 was passed through the column and fractionated with the aforementioned column buffer. Fractions (4 ml.) were collected using an ISCO (Lincoln, Neb.) fraction collector. Samples were assayed for protein by monitoring A280 nm. Sixteen pools of the 4 ml fractions were lyophilized, and then reconstituted in 2 ml. of deionized water, except for fraction 7 which was reconstituted with 15 ml. of water to facilitate dissolution.

Binding assays on each fraction were carried out as follows. To 0.5 ml of each fraction (Tube no. 1) was added 25 µl of $[^3H]$-dihydrocyclosporine. To an identical sample (Tube no. 2) was added the same amount of tracer, plus 64 ng of unlabeled cyclosporine. After incubating the binding mixtures for 12 hours at 37° C, bound and free tracers were separated by the procedure described in Example 2. Specific binding was calculated as described above for both samples.

In the histogram of Fig. 5 (Graph A) is plotted the amount of radioactive $[^3H]$-dihydrocyclosporine bound to protein, in the absence (Tube no. 1) and presence (Tube no. 2) of competing unlabeled cyclosporine as a function of the average molecular weight of each of the column fractions. The differences between Tubes 1 and 2, which reflect binding competition, for each column fraction are plotted as a function of the average

molecular weight of each column fraction in Fig. 6. It is clear that a cytosolic fraction representing one or more proteins of average molecular weight of approximately 10,250 dal. was most active in the competitive protein binding assay of the invention, and that the 17,000 dal. and 2,650 dal. fractions also exhibited acceptable competitive cyclosporin A binding activity.

EXAMPLE 5

Analysis of Cyclosporine in Patient Blood Samples: Comparison of Competitive Protein Binding Assay with HPLC Analysis

Sixteen blood samples from transplantation patients receiving CsA were analyzed in duplicate - by a competitive protein binding assay carried out in accordance with this invention and by an HPLC procedure.

For the binding assay, the procedures of Example 3 were followed, using [$^3$H]-dihydrocyclosporine as the tracer, but using a 1.5 hr. incubation period.

HPLC analysis was carried out according to the procedure of Giesbrecht [Giesbrecht, E.E., et al., Therap. Drug Monitoring, in press 1988]. Briefly, a protein-free filtrate of patient blood was prepared by extracting 250 µl of whole blood with 750 µl of acetonitrile: methanol (9:1) containing the internal standard CsD. After centrifuging the mixture, the supernatant fluid was passed through cartridges of C18 and Silica Bond Elut to remove impurities. Eluates were dried, reconstituted in acetonitrile: water (1:1), then extracted with heptane to remove lateeluting peaks. HPLC was performed at 75°C using a SUPELCOSIL™ C18 column (10 cm x 4.6 mm) with 3 µ packing (Supelco, Inc.) using a Waters Scientific Co. HPLC system. The mobile phase of acetonitrile: phosphate buffer, pH 2.5 (77:23) was run at a flow rate of 0.6 ml/min. Absorbancy of the eluted fractions was estimated at 214 nm. Recovery for CsA was 92-104%, at a lower sensitivity of 15 µg/L. The method was linear to 500 µg CsA/L. Peak height measurements were compared to CsA external standards of 150 and 400 µg/L.

The results are shown in Fig. 7, wherein the results of each HPLC assay for CsA (in µg/L) is plotted against the result for the same sample obtained by the competitive protein binding assay of the invention (RRA, µg/L). Different scales were required as HPLC yield values substantially lower than did the RRA. For example, patient samples that were approximately 500 µg/L by HPLC ranged from 110 to 1600 µg/L by the RRA. Such results indicate that the RRA estimated not only the parent CsA, but metabolites as well. The fact that these metabolites reacted with a cytosolic binding proteins suggests that these molecules are biologically active, and suggests further that the binding assay of the invention is capable of estimating all pharmacologically active species of cyclosporine.

The above discussion of this invention is directed primarily to preferred embodiments and practices thereof. It will be readily apparent to those skilled in the art that further changes and modifications in the actual implementation of the concepts described herein can easily be made without departing from the spirit and scope of the invention as defined by the following claims.

## Claims

1. A water-soluble macromolecular binding protein for cyclosporine and biologically-active derivatives, analogues and metabolites thereof preparable by a process comprising the steps of:

(a) centrifuging homogenized, disrupted, normal or transformed cells to produce water-soluble proteins;

(b) fractionating said water-soluble proteins;

(c) testing the thus-obtained protein fractions for cyclosporine binding activity; and

(d) selecting those protein fractions having acceptable cyclosporine binding activity.

2. A binding protein as claimed in claim 1 wherein said cells comprise: human or animal, peripheral or cultured, white blood cells or tissue precursors thereof; human or animal erythrocytes or tissue precursors thereof; human or animal, tissue or cultured reticulcendothelial cells; human or animal, tissue or cultured, liver, spleen, bone marrow or thymus cells; or arthropod, trematode, mollusc, fungi, porifera, yeast or bacterial cells.

3. A binding protein as claimed in claim 1 or 2 wherein said centrifuging employs forces of at least

20,000 x g for at least 15 minutes at a temperature below 25° C.

4. A binding. protein as claimed in claim 1, 2 or 3 wherein said water-soluble proteins are fractionated by a method that comprises filtering said water-soluble proteins through one or more size exclusion gel columns with a fractionation range from between about 2,000 and 150,000 daltons or through one or more molecular weight cut off membrane filters.

5. A binding protein as claimed in any one of claims 1 to 4, wherein those of the tested protein fractions having acceptable cyclosporine binding activity are selected by a method comprising applying a competitive protein binding assay wherein labeled cyclosporin A competes with unlabeled cyclosporin A for binding sites on said binding protein to each fraction.

6. A binding protein as claimed in any one of claims 1 to 5 having a molecular weight in the range of: 2,400 to 3,000 daltons; 8,000 to 13,500 daltons; or 13,500 to 25,000 daltons.

7. A competitive protein binding assay for cyclosporine (or a biologically active derivative, metabolite or analogue thereof), in which a labeled cyclosporine (or biologically active derivative, metabolite or analogue thereof) competes with analyte for binding to a water-soluble binding protein as claimed in any one of claims 1 to 6.

8. A quantitative competitive protein binding assay for cyclosporine and biologically-active derivatives, analogues and metabolites thereof contained in an initial fluid sample comprising the steps of:

(a) preparing an extract of said initial fluid sample, said extract containing said cyclosporines;

(b) evaporating an aliquot of said extract to dryness;

(c) preparing an aqueous solution of said dried aliquot and a water-soluble binding protein as claimed in any one of claims 1 to 6;

(d) adding to said aqueous solution radioactive cyclosporin A and incubating the resulting reaction mixture until steady state binding is reached;

(e) physically separating protein-bound labeled cyclosporin A from unbound labeled cyclosporin A;

(f) quantifying the amount of labeled cyclosporin A specifically bound to protein;

(g) determining the amount of cyclosporine and biologically-active derivatives, analogues and metabolites thereof in said initial fluid sample by referring the quantified amount of labeled cyclosporin A specifically bound to protein obtained in step (f) hereinabove to a standard curve prepared by analyzing a series of cyclosporine standard by solutions the competitive protein binding assay of steps (a) - (f), inclusive, hereinabove, and 34 plotting;

$[B_{(std)}\text{-NSB}/B_{(0\ std)}\text{-NSB}]100$ against log [Cs]

wherein $B_{(std)}$ is the amount of cyclosporine bound to the binding protein, $B_{(0\ std)}$ is a control, NSB is the non-specific binding of each sample of cyclosporine, and [Cs] is the concentration of standard cyclosporine assayed.

9. A method as claimed in claim 8 wherein said extract is prepared by extracting cyclosporines, analogues, derivatives and metabolites from initial fluid samples using an alcohol selected from $C_1$ to $C_6$ straight or branched chain, primary, secondary and tertiary alkanols.

10. A method as claimed in claim 8 or 9 wherein said radioactive cyclosporin A is $^3$H-labeled cyclosporin A or $^{125}$I-labeled cyclosporin A.

11. A method as claimed in claim 8, 9 or 10 wherein steady state binding is reached in a period ranging from 0.5 to 16 hours and at a temperature of from 25° C to 37° C.

12. A method as claimed in any one of claims 8 to 11 wherein physical separation of protein-bound and unbound labeled cyclosporin A is accomplished by a method comprising: filtration using a filter selected from glass fibre filters, nitrocellulose filters and polymeric filters; particulate adsorption, for example wherein the adsorbent is selected from the group consisting of protein-coated charcoal and dextran- coated charcoal; protein precipitation; or filtration using a size exclusion column.

13. A quantitative competitive protein binding assay for cyclosporine and biologically-active derivatives, analogues and metabolites thereof contained in an initial fluid sample comprising the steps of:

(a) preparing an extract of said initial fluid sample, said extract containing said cyclosporines;

(b) preparing an aqueous solution of said extract and a water-soluble binding protein as claimed in any one of claims 1 to 6;

(c) adding to said aqueous solution a fluorophore-labeled cyclosporin A and incubating the resulting mixture until steady state binding is reached;

(d) measuring the fluorescent polarization signal of the thus-incubated mixture, and converting said fluorescent polarization signal to an amount of protein-bound, fluorophore-labeled cyclosporin A; and

(e) determining the concentration of cyclosporine and biologically-active derivatives, analogues and metabolites thereof in said initial fluid sample by reference to a fluorescence polarization standard curve obtained by analyzing a series of cyclosporine standard solutions by said fluorescence polarization

competitive protein binding assay, and plotting
$[B_{(std)}/B_{(0\ std)}]$ 100 against log [Cs]
wherein the terms $B_{(std)}$, $B_{(std)}$ and [Cs] are as defined in claim 8.

14. A method as claimed in claim 13 wherein said fluorophore is fluorescein or luciferin.

15. A quantitative competitive protein binding assay for cyclosporine and biologically-active derivatives, analogues and metabolites thereof contained in an initial fluid sample, comprising the steps of:

(a) preparing an extract of said initial fluid sample, said extract containing said cyclosporines;

(b) evaporating an aliquot of said extract to dryness;

(c) preparing an aqueous solution of said dried aliquot and a water-soluble binding protein as claimed in any one of claims 1 to 6;

(d) adding to said aqueous solution enzyme-conjugated cyclosporin A and incubating the resulting reaction mixture until steady state binding is reached;

(e) physically separating protein-bound enzyme-conjugated cyclosporin A from unbound enzyme-conjugated cyclosporin A;

(f) quantifying the amount of enzyme-conjugated cyclosporin A specifically bound to protein; and

(g) determining the amount of cyclosporine and biologically-active derivatives, analogues and metabolites thereof in said initial fluid sample by referring the quantified amount of enzyme-labeled cyclosporin A bound to protein obtained in step (f) hereinabove to a standard curve prepared by analyzing a series of cyclosporine standard solutions by the competitive protein binding assay of steps (a)-(f), hereinabove, and plotting
$[B_{(std)}\text{-NSB}/B_{(0\ std)}\text{-NSB}]$100 against log [Cs]
wherein the terms B(std), NSB, B(0 std) [Cs] are as defined in claim 8.

16. A method as claimed in claim 15 wherein quantifying in steps (f) and (g) is carried out using chemiluminescent methods, such as, for example, wherein said enzyme is alkaline phosphatase and the chemiluminescent substrate is a water-soluble 1,2-dioxetane decomposible by said enzyme to yield light energy or, for a second example, wherein said enzyme is an $\alpha$- or $\beta$-galactosidase and the chemiluminescent substrate is a water-soluble 1,2-dioxetane decomposible by said enzyme to yield light energy.

17. A binding assay as claimed in any one of claims 7 to 16 which is carried out in a solid state.

18. A kit for a quantitative competitive protein binding assay for cyclosporine and biologically-active analogues, derivatives and metabolites thereof in an initial fluid sample comprising:

(a) a mixture of binding protein of claim 1 and a protein stabilizer;

(b) labeled cyclosporine;

(c) cyclosporine standards; and

(d) a mixture comprising a buffer and surfactant stabilizer.

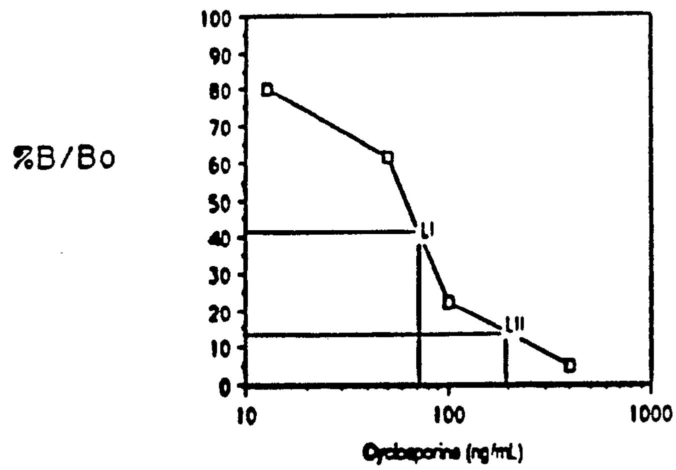


FIGURE 1: %B/Bo VS CYCLOSPORINE COMPETITIVE BINDING ASSAY CURVE. CONSTANT CYTOSOL PREPARATION AND CONSTANT 3H DIHYDROCYCLOSPORINE INCUBATED AT 37° WITH DOUBLING CONCENTRATIONS OF CYCLOSPORINE STANDARD. BOUND FRACTION WAS SEPARATED FROM FREE USING GLASS FILTERS.

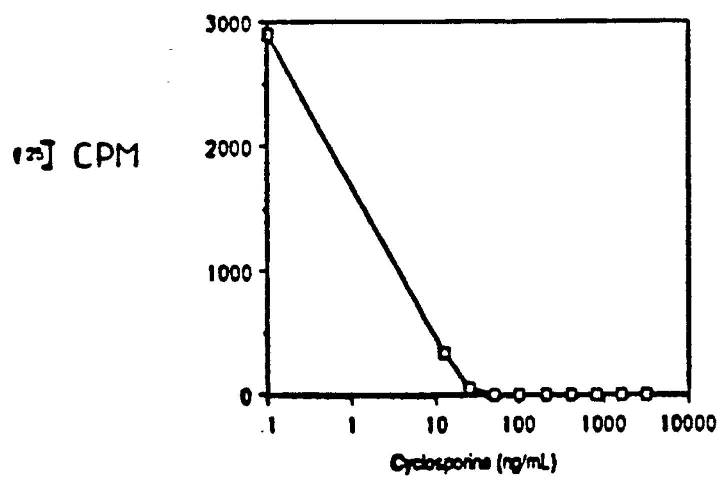


FIGURE 2: CPM $^{25}$I VS CYCLOSPORINE COMPETITIVE BINDING ASSAY CURVE. TEST CONDITIONS WERE THE SAME AS FIGURE 1. THE CYCLOSPORINE STANDARD WHICH REPRESENTS 12 NG/ML IN WHOLE BLOOD HAS A BOUND VALUE OF 12%. 25 NG/ML HAS A BOUND VALUE OF 2%. THE STRIKING BINDING DIFFERENCE IMPLIES A LOWER AFFINITY TO THE RECEPTOR SITES. THIS ALLOWS FOR GREATER ANALYTICAL SENSITIVITY. BOUND FRACTION SEPARATED WITH GLASS FILTERS.

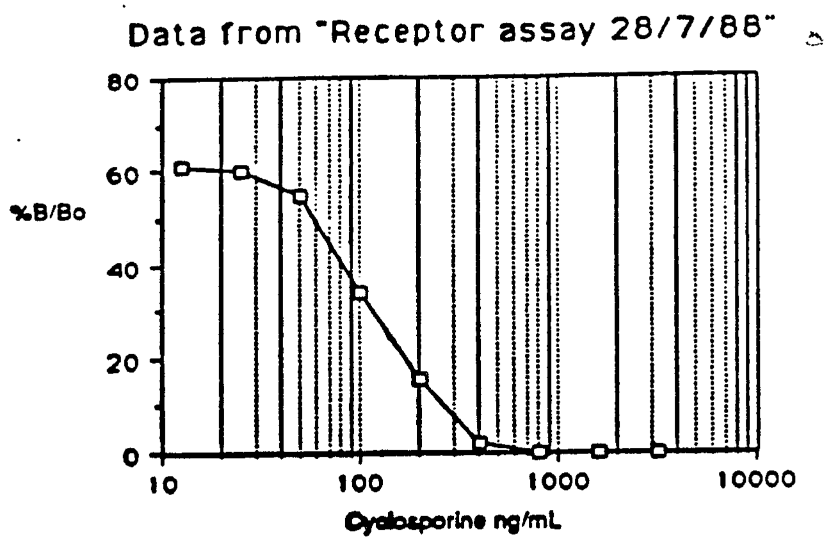


FIGURE 3. % B/Bo VS CYCLOSPORINE STANDARDS COMPETITIVE BINDING ASSAY. $^3$H DIHYDROCYCLOSPORINE AND CYTOSOL RECEPTOR UNDER CONSTANT CONDITIONS WITH INCREASING CONCENTRATIONS OF CYCLOSPORINE STANDARDS. BOUND FRACTION WAS SEPARATED USING GLASS FILTERS.

Fig. 4

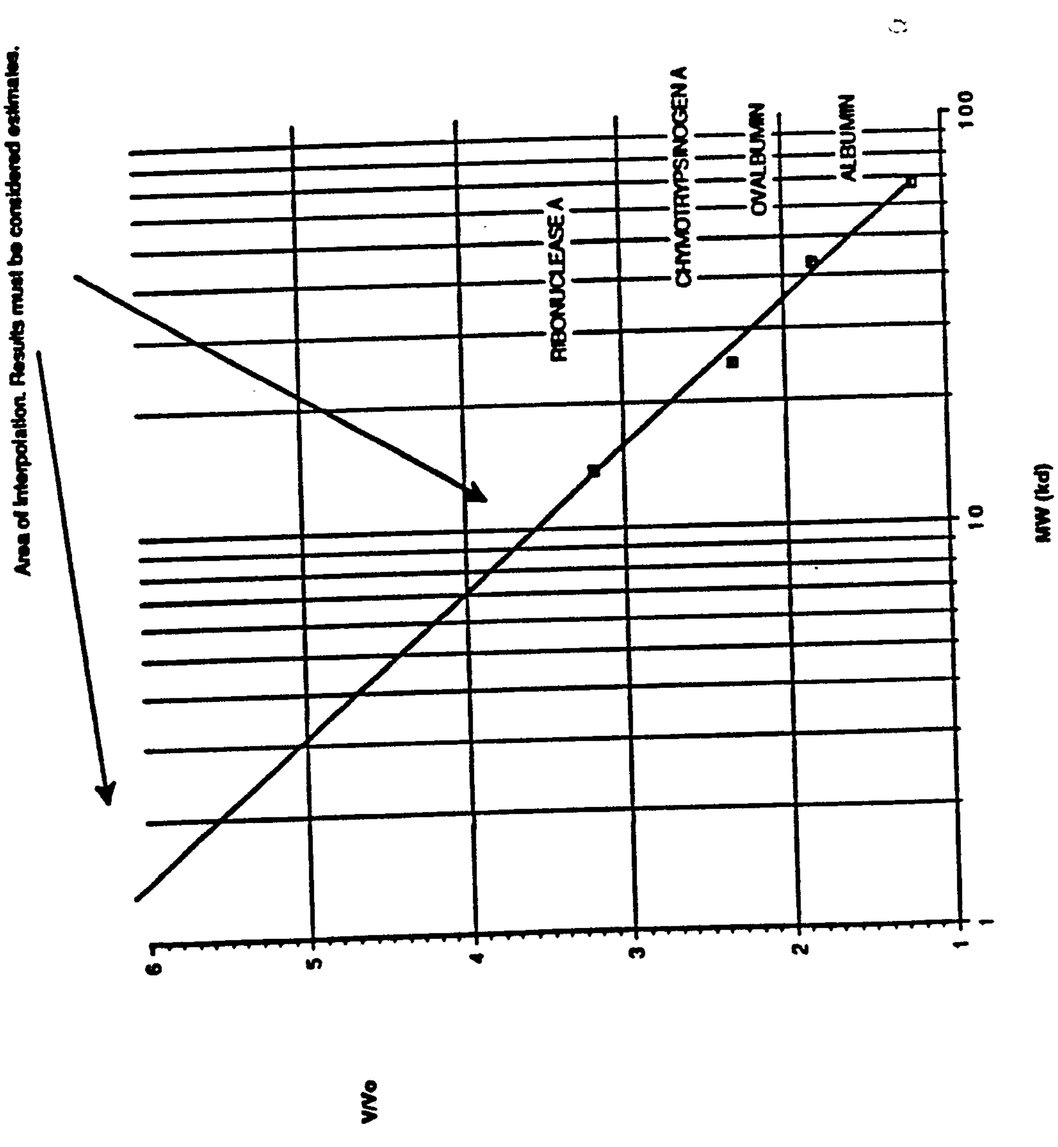
Area of Interpolation. Results must be considered estimates.
RIBONUCLEASE A
CHYMOTRYPSINOGEN A
OVALBUMIN
ALBUMIN
V/Vo
6
5
4
3
2
1
MW (kd)
1
10
100

Fig. 5

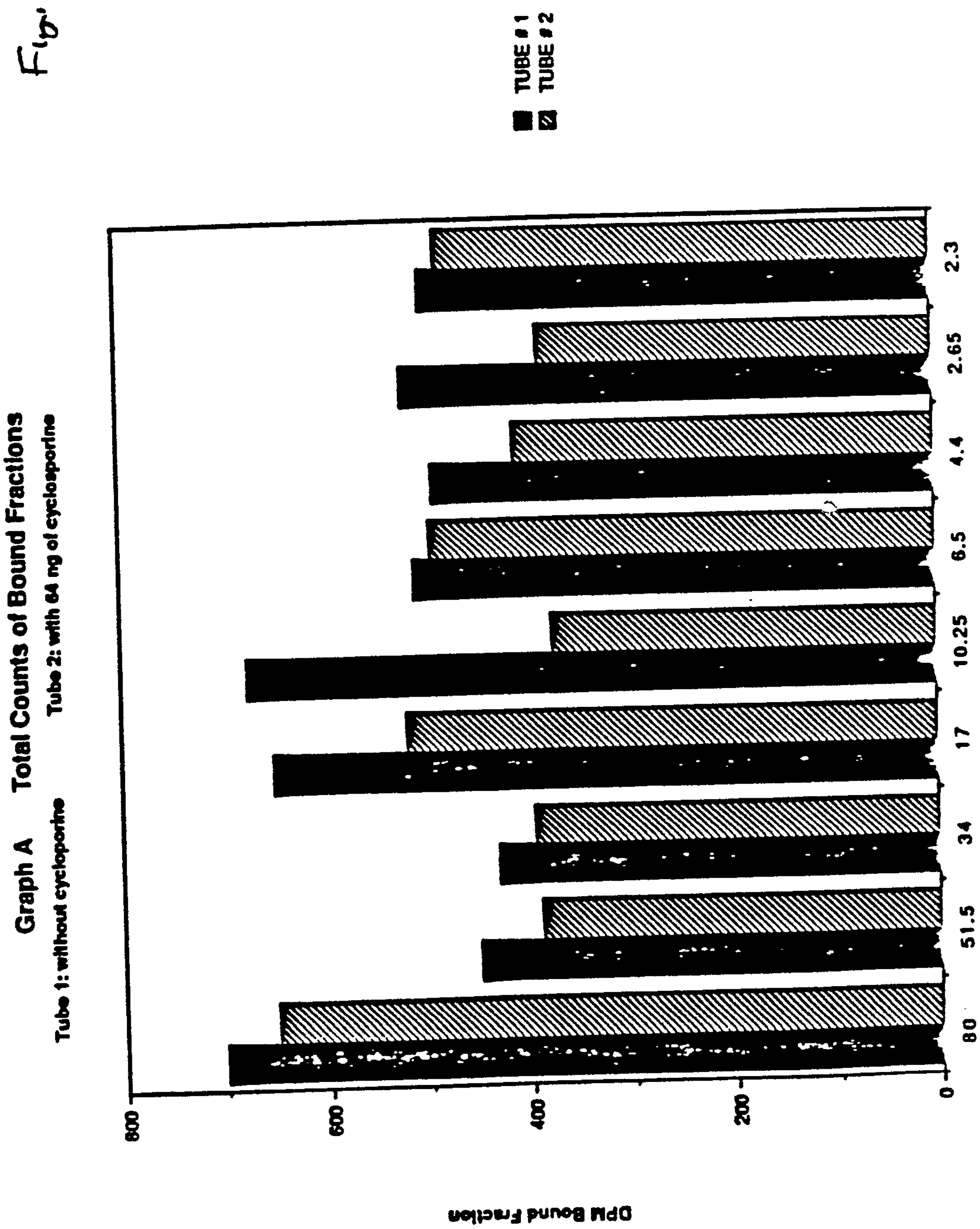
Graph A
Total Counts of Bound Fractions
Tube 1: without cycloporine
Tube 2: with 64 ng of cyclosporine
TUBE # 1
TUBE # 2
DPM Bound Fraction
800
600
400
200
0
80
51.5
34
17
10.25
6.5
4.4
2.65
2.3
Average Molecular Weight (kd)

Fig. 6

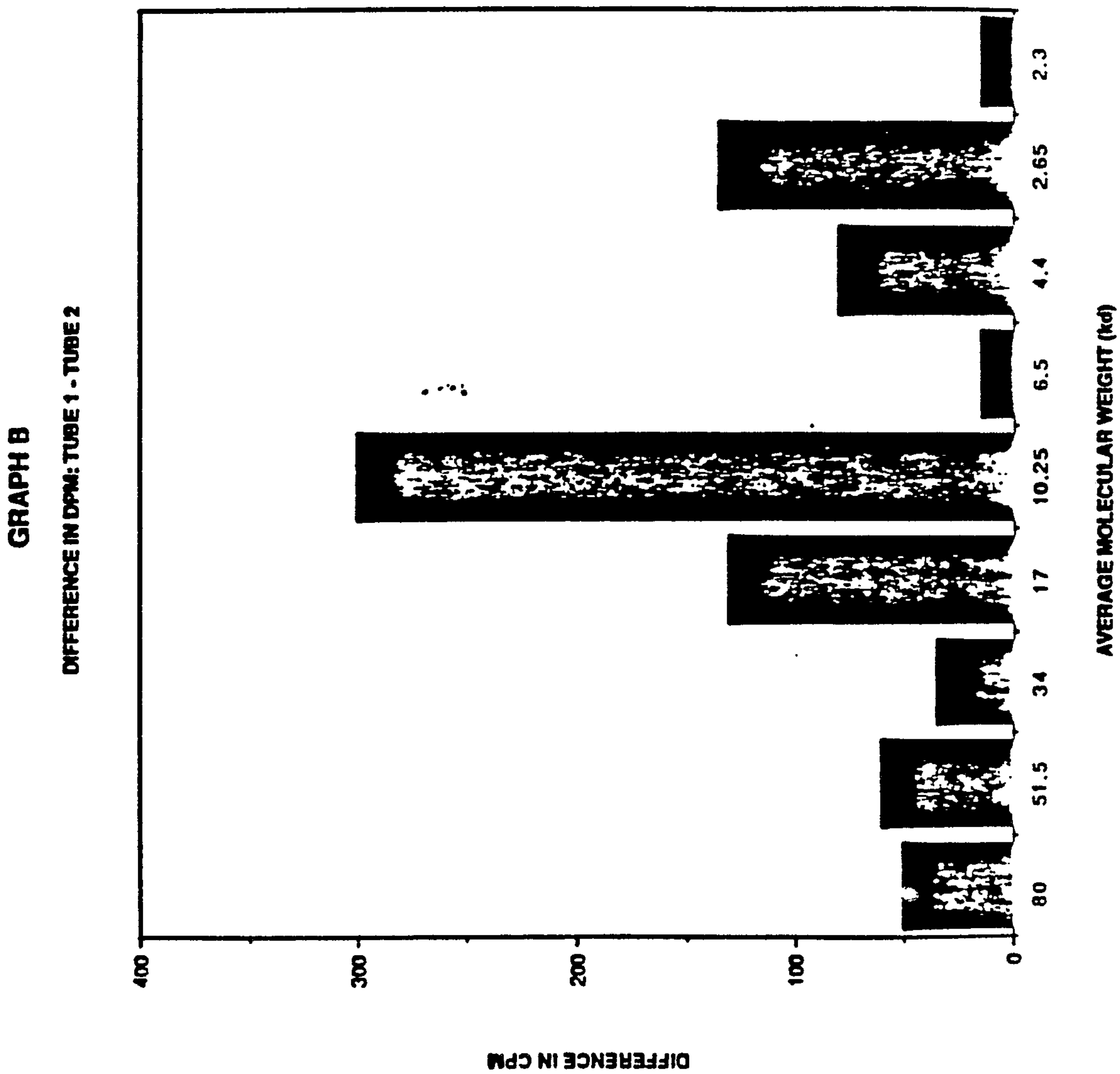
GRAPH B
DIFFERENCE IN DPM: TUBE 1 - TUBE 2
DIFFERENCE IN CPM
0
100
200
300
400
80
51.5
34
17
10.25
6.5
4.4
2.65
2.3
AVERAGE MOLECULAR WEIGHT (kd)

Fig. 7

WHOLE BLOOD ANALYSIS

HPLC VS RRA

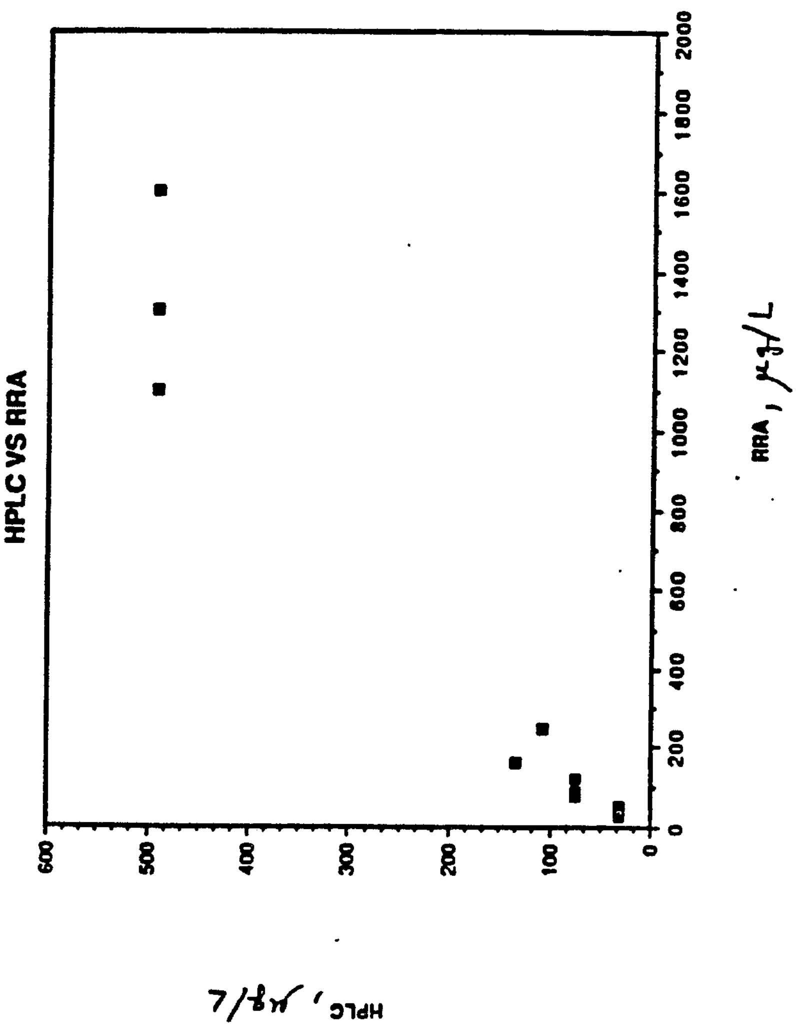


37° INCUBATION

BOUND SEPARATED BY PROCEDURE A

N=16